# EUROPEAN PATENT APPLICATION

(11) **EP 4 350 329 A1**
(43) Date of publication of application: **10.04.2024**
(21) Application number: 23202187.3
(22) Date of filing: 06.10.2023
(51) Int. Cl.: G01N 19/04, G01N 13/00, G01N 33/00

(54) **METHODS FOR QUANTIFICATION OF SOLVENT-SUBSTRATE INTERACTIONS**

(30) Priority: 06.10.2022 EP 22199932
(71) Applicant: The Procter & Gamble Company, Cincinnati, OH 45202 (US)
(72) Inventor: ZONFRILLI, Fabio, 1853 Strombeek-Bever, Brussels (BE); GABRIELE, Andrea, 1853 Strombeek-Bever (BE)
(74) Representative: P&G Patent Belgium UK

(57) **Abstract**

Provided herein is a method and system for a quantification of solvent-substrate interaction parameters of a solvent (160) and a substrate sample (108) interaction, the substrate sample (108) having a first surface (106), an opposing second surface (104) and a straight side edge (102) connecting the first surface (106) and the second surface (104), comprising:
- contacting the straight side edge (102) of the substrate sample (108) with a solvent (160),
- measuring, from the substrate sample (108), a set of solvent expansion parameters comprising:
- a swelling rate of the substrate sample (108), and
- a penetration rate of the solvent (160) into the substrate sample (108) resulting from the contacting,

wherein the set of solvent expansion parameters is determined from an image dataset of at least a portion of the straight side edge (102), each optical image (200, a, b) captured at a different time points during the contacting.

## Description

### Field of the invention

The present invention is in a field of quantification of solvent-substrate interactions, and, systems and computer programs therefor.

### Background to the invention

Interactions between solvents and substrates are important for many aspects of product behaviour. For instance, a substrate that is an elastomeric gel is found in foods and medicines, and also in important and diverse applications including valves for microfluidic devices, and tissue engineering. A substrate that is a wet adhesion film is used as packaging of different substances including detergents, food products, pharmaceuticals and agricultural chemicals. Typically a portion of the film is wetted and the wetted portion is brought into contact with another substrate, thereby forming a joint between the film and substrate in order to form a part of a product that is container or pouch for holding the substance.

Characterising how a substrate behaves in the presence of solvent is important in prediction of product behaviour, for instance, in response to applied mechanical deformation and forced permeation.

Methods for quickly and reproducibly measuring useful parameters are needed, in particular, methods that rapidly characterise the interactions by allowing multiple parameters to be determined from a single measurement. This open up a possibility for high throughput screening of different substrates. The reproducibly allows the same substate to be compared at different times and locations, without significant deviation for the same substate. The reproducibly further allows reliable detection of variations between different batches of substrate.

The present disclosures provide methods to rapidly and reproducibly quantify different parameters of solvent-substrate interactions.

### Summary of the invention

Provided herein is a method for a quantification of solvent-substrate interaction parameters of a solvent (160) and a substrate sample (108) interaction, the substrate sample (108) having a first surface (106), an opposing second surface (104) and a straight side edge (102) connecting the first surface (106) and the second surface (104), comprising:
- contacting the straight side edge (102) of the substrate sample (108) with a solvent (160),
- measuring, from the substrate sample (108), a set of solvent expansion parameters comprising:
   - **a** swelling rate of the substrate sample (108), and
   - a penetration rate of the solvent (160) into the substrate sample (108) resulting from the contacting,

wherein the set of solvent expansion parameters is determined by:
   - generating an image dataset comprising a plurality of optical images (200, a,b,c) of at least a portion of the straight side edge (102), each optical image (200, a, b) captured at a different time point during the contacting;
   - identifying in each optical image (200, a,b,c) or from an image portion thereof (200', a,b,c) a first front edge (202, a,b,c) corresponding to the straight side edge (102), and a second front edge (262, a,b,c) corresponding to a front edge (162) of the solvent (160) penetrating the substrate sample (108); and
   - the swelling rate is determined from a position (p1) of the first front edge (202, a,b,c) over the different timepoints, and the penetration rate is determined from a position (p2) of the second front edge (262a,b,c) over the different timepoints,
wherein the solvent-substrate interaction parameters are determined from the set of solvent expansion parameters.

Preferably, the first (202, a,b) and second (262, a,b) front-edges may be identified comprising the steps:
- for each optical image (200, a,b) in the image dataset, or image portion thereof (200', a,b):
   - with the first front edge (202, a,b,c) aligned parallel to a y-axis, and an x-axis being perpendicular to the y-axis;
   - applying a first filter to the optical image (200, a,b) or image portion (200',a,b), comprising replacing (C1' to C4') each pixel of each column (C1 to C4) of pixels parallel to the y-axis into an average of grey intensity for that column;
   - optionally applying one or more edge-enhancing filters to each previously-filtered image;
   - thereby obtaining an enhanced image (300, a,b) or enhanced image portion (300',a,b),
   - identifying from the enhanced image (300, a,b) or enhanced image portion (300',a,b) thereof, an enhanced first region (302, a,b) and enhanced second region (362,a,b), the enhanced first (302, a,b) and second (362, a,b,c) regions have the highest grey intensities compared with other regions, wherein the enhanced first region (302, a,b) corresponds to the first front edge (202, a,b) and the enhanced second region (362,a,b) corresponds to the second front edge (262, a,b).

Preferably, the identifying from enhanced image (300, a,b) the first front edge (222,a, b) and second front edge (262,a, b)) further comprises the steps:
- for each enhanced image (300, a,b) or enhanced image portion (300', a,b)
- determining an intensity profile (400,a, b) along the x-axis of the enhanced image (302,a,b), wherein intensity profile (400,a, b) indicates along one axis pixel intensity of the enhanced image (302,a,b) or enhanced image portion (300', a,b) as an amplitude, and along another axis position along the x-axis of the enhanced image (302,a,b) or enhanced image portion (300', a,b),
- optionally applying one or more noise-reducing filters to the intensity profile (400,a, b)
- identifying in the intensity profile (400a, b), optionally noise-filtered, a first peak (402,a,b) and a second peak (462,a,b) have the highest maximum amplitudes compared with other regions, wherein the first peak (402,a,b) corresponds to the first front edge (202, a,b) and the second peak (462,a,b) corresponds to the second front edge (262a,b,c).

Preferably:
- the swelling rate is represented as a first exponential function fitted to the position (*p1*) of the first front edge (202, a,b,c) over the different timepoints,
- the penetration rate is represented as a second exponential function fitted to the position (*p2*) of the second front edge (262, a,b,c) over the different timepoints.

Preferably the substrate sample (108) comprises superabsorbent polymer (SAP or AGM), acrylonitrile, butadiene styrene (ABS), polybutadiene (*e.g.* butadiene rubber, BR), polycarbonates (PC), polyethersulfones (PES), polyethylene terephthalate (PET), poly(methyl methacrylate) (PMMA), polystyrene (PS), polysulfones (PSU), polyvinyl chloride (PVC), cellulose, or polyvinylalcohol (PVOH), or a combination or two or more of the aforementioned polymers.

Further provided is a computer-implemented method for a quantification of solvent-substrate interaction parameters of a solvent (160) and a substrate sample (108) interaction, the method comprising:
- receiving an image dataset comprising a plurality of optical images (200, a,b,c) of at least a portion of a straight side edge (102) of the substrate sample (108), each optical image (200, a,b,c) captured at a different time point during contacting the straight side edge (102) of the substrate sample (108) with an solvent (160),
- identifying in each optical image (200, a,b,c) or an image portion (200', a,b,c) thereof a first front edge (202, a,b,c) corresponding to the straight side edge (102), and a second front edge (262a,b,c) corresponding to a solvent front (162) of the solvent (160) penetrating the substrate sample (108); and
- determining a set of expansion parameters comprising:
   - a swelling rate of the substrate sample, and
   - a penetration rate of the solvent (160) into the substrate sample (108),
      wherein the swelling rate is determined from a change in position (*p1*) of the first front edge (202, a,b,c) over the different timepoints, and the penetration rate is determined from a change in position (*p2*) of the second front edge (262a,b,c) over the different timepoints,
- determining from the set of solvent expansion parameters the solvent-substrate interaction parameters.

The computer implemented method may incorporate the subject matter described herein.

Further provided is a system comprising a processor adapted to perform the computer-implemented method described herein.

Further provided is a computer-implemented method for determining a quantification of solvent-substrate interaction parameters of a solvent (160) and a substrate sample (108) interaction, the method comprising:
- sending, to a remote processor, an image dataset comprising a plurality of optical images (200, a,b,c) of at least a portion of a straight side edge (102) of the substrate sample (108), each optical image (200, a,b,c) captured at a different time point during contacting the straight side edge (102) of the substrate sample (108) with an solvent (160);
- receiving from the remote processor, the quantification of solvent-substrate interaction parameters, wherein the quantification of solvent-substrate interaction parameters has been determined by:
   - identifying in each optical image (200, a,b,c) a first front edge (202, a,b,c) corresponding to the straight side edge (102), and a second front edge (262a,b,c) corresponding to a solvent front (162) of the solvent (160) penetrating the substrate sample (108); and
   - determining a set of expansion parameters comprising:
      - a swelling rate of the substrate sample (108), and
      - a penetration rate of the solvent (160) into the substrate sample (108), wherein the swelling rate is determined from a change in position (*p1*) of the first front edge (202, a,b,c) over the different timepoints, and the penetration rate is determined from a change in position (*p2*) of the second front edge (262a,b,c) over the different timepoints,
   - determining from the expansion parameters, the quantification of solvent-substrate interaction parameters.

The computer implemented method may incorporate the subject matter described herein.

Further provided is a system comprising a processor adapted to perform the computer-implemented method described herein.

### Figure Legends

**FIG. 1A** is a three-dimensional schematic view of a substrate sample.
**FIG. 1B** is a plan schematic view of the substrate sample of FIG. 1A
**FIG. 2** is a schematic view the substrate sample and solvent prior to mutual contacting.

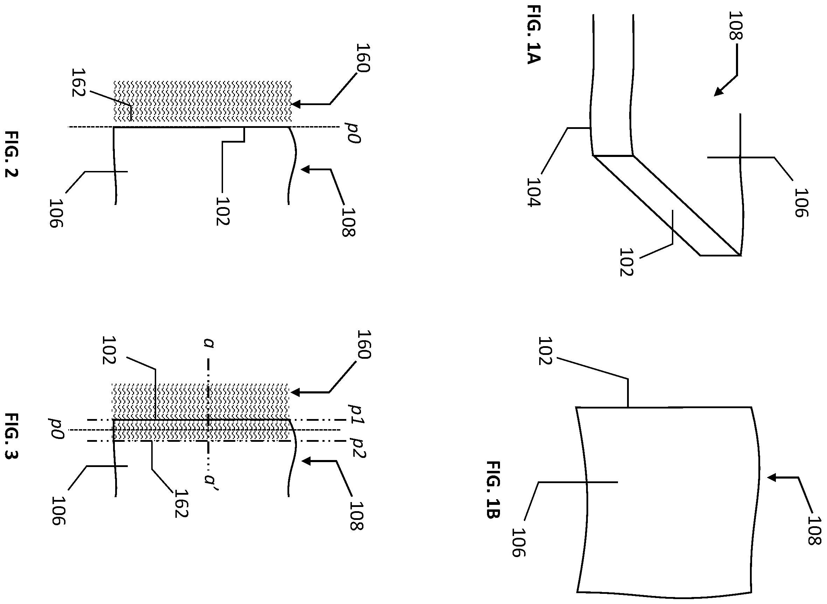

**FIG. 2** is a schematic view the substrate sample and solvent after mutual contacting, substrate swelling and solvent penetration.
**FIG. 3** is a schematic view the substrate sample and solvent after mutual contacting, substrate swelling and solvent penetration.

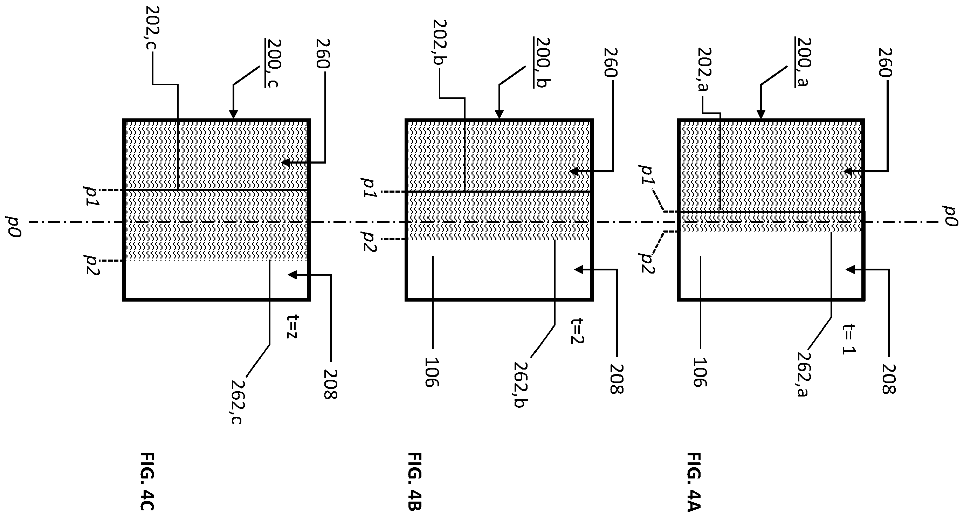

**FIGs. 4A** to **4C** are a schematic views of optical images and image portions there at different times during contacting (FIG. 4A t=1; FIG. 4B t=2; FIG. 4C t=z;).

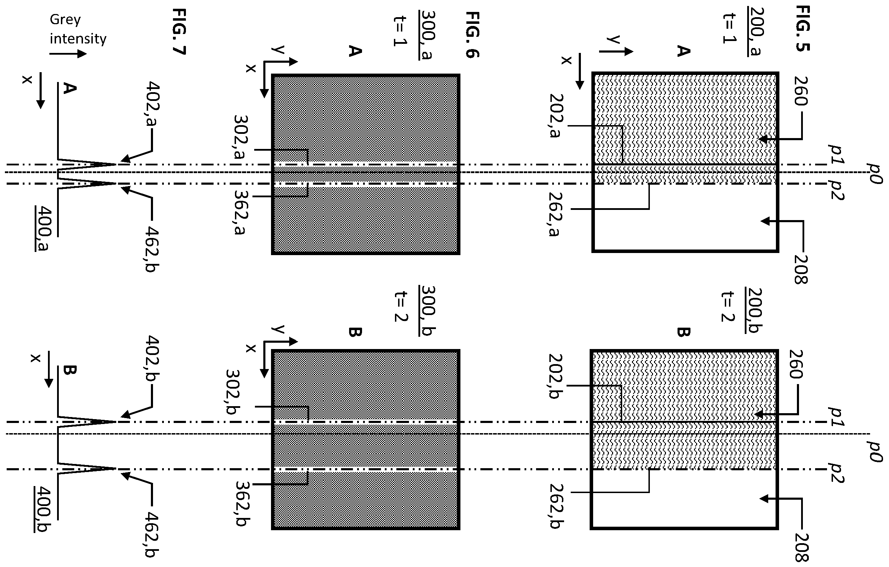

**FIGs. 5A** and **5B** are a schematic views of optical images at different times during contacting (FIG. 5A t=1; FIG. 5B t=2).
**FIGs. 6A** and **6B** are a schematic views of enhanced optical images at different times during contacting (FIG. 6A t=1; FIG. 6B t=2).
**FIGs. 7A** and **7B** are examples of intensity profiles at different times during contacting (FIG. 7A t=1; FIG. 7B t=2).

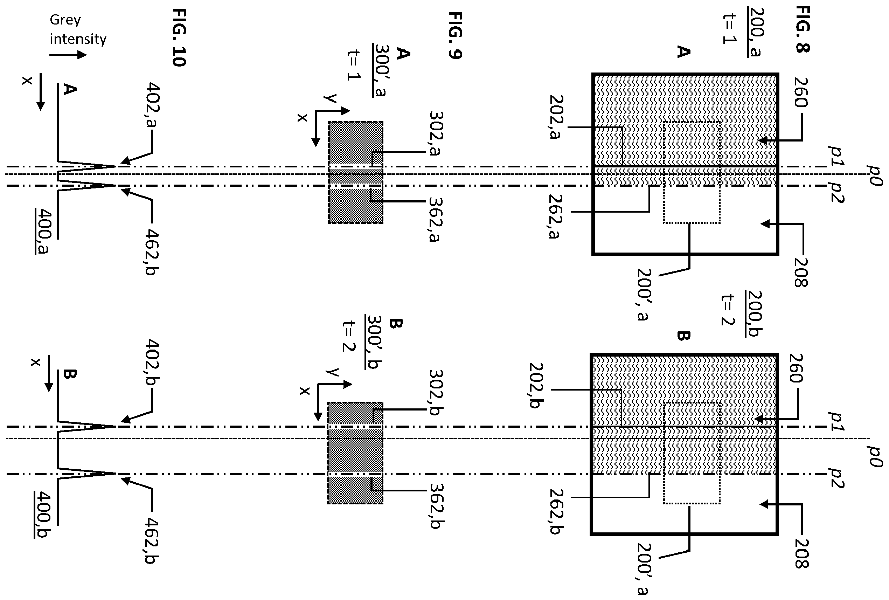

**FIGs. 8A** and **8B** are a schematic views of optical images and optical image portions thereof at different times during contacting (FIG. 5A t=1; FIG. 5B t=2).
**FIGs. 9A** and **9B** are a schematic views of optical image portions at different times during contacting (FIG. 6A t=1; FIG. 6B t=2).
**FIGs. 10A** and **10B** are examples of intensity profiles at different times during contacting (FIG. 7A t=1; FIG. 7B t=2).

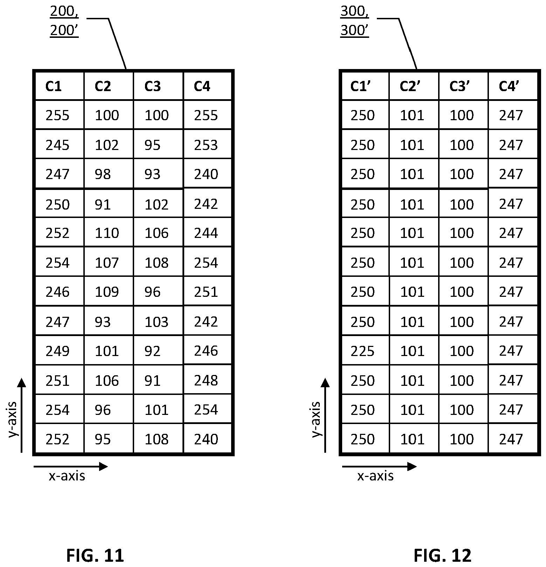

**FIG. 11** is a schematic pixel grey intensity representation of an optical image or optical image portion.
**FIG. 12** is a schematic pixel grey intensity representation of an enhanced optical image or enhanced optical image portion from averaging pixel column of FIG. 11.

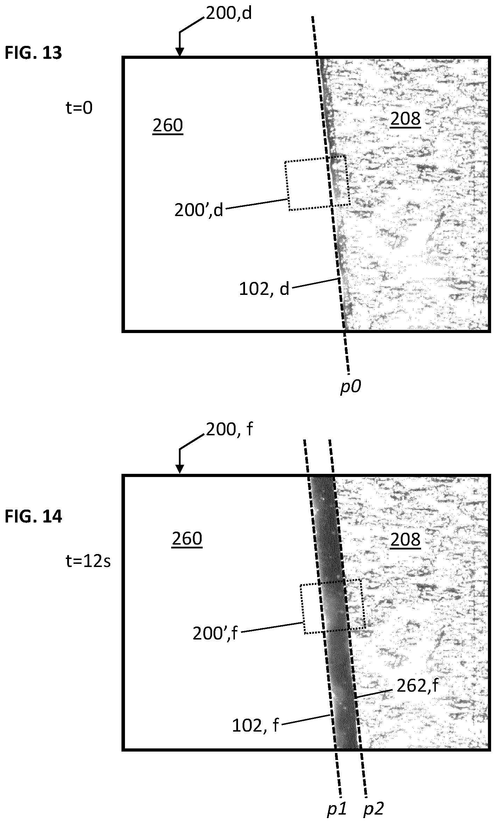

**FIG. 13** shows an optical image and optical image portion thereof of the substrate sample prior to contacting with solvent.
**FIG. 14** shows an optical image and optical image portion thereof of the substrate sample during contacting, substrate swelling and solvent penetration has completed.

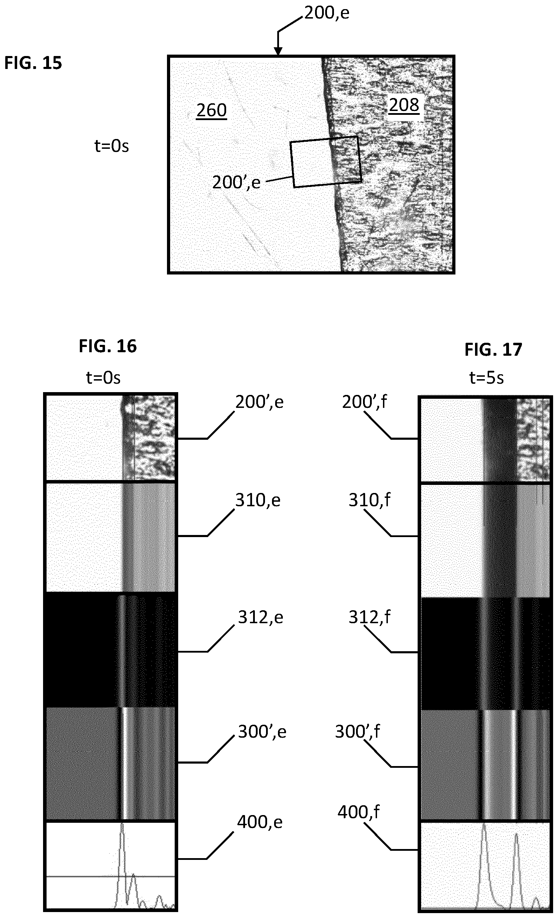

**FIG. 15** shows an optical image and optical image portion thereof of the substrate sample prior to contacting with solvent.
**FIG. 16** shows in alignment from top to bottom: optical image portion of the substrate sample of
FIG 15, result of applying the first filter, result of subsequently applying an edge-enhancing (Sobel) filter, result of subsequently applying an edge-enhancing (Laplace) filter, an intensity profile.
**FIG. 17** shows in alignment from top to bottom: optical image portion of the substrate sample of FIG. 15/16 after contacting with solvent for 5 seconds, result of applying the first filter, result of subsequently applying an edge-enhancing (Sobel) filter, result of subsequently applying an edge-enhancing (Laplace) filter, an intensity profile wherein noise reducing filters have been applied to the intensity profile.

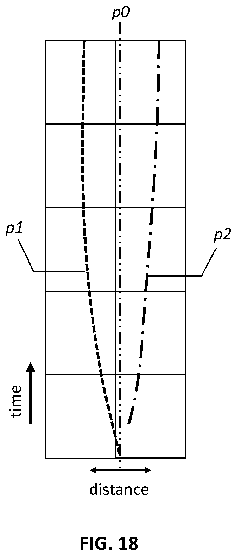

**FIG. 18** show a plot of position (*p1*) (first peak or straight side edge) and position (*p2*) (second peak or solvent front) at different time points.

### Detailed description of invention

Before the present methods, computer programs and systems of the invention are described, it is to be understood that this invention is not limited to particular methods, computer programs and systems or combinations described, since such methods, computer programs and systems and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. Unless otherwise indicated, all figures and drawings in this document are not to scale and are chosen for the purpose of illustrating different embodiments of the invention. In particular the dimensions of the various components are depicted in illustrative terms only, and no relationship between the dimensions of the various components should be inferred from the drawings, unless so indicated.

It is to be understood that other embodiments may be utilised and structural or logical changes may be made without departing from the scope of the present invention. The following detailed description, therefore, is not to be taken in a limiting sense, and the scope of the present invention is defined by the appended claims.

The present invention concerns a method for quantification of solvent-substrate interaction parameters of an interaction between a solvent and a substrate sample (108). An exemplary substrate sample (108) is depicted in **FIGs. 1A** and **1B**. The substrate sample (108) has a first surface (106), an opposing second surface (104) and a straight side edge (102) connecting the first surface (106) and the second surface (104).

The method comprises contacting the straight side edge (102) of the substrate sample with an solvent (160) and measuring, from the substrate sample (108), a set of expansion parameters. The expansion parameters comprise a swelling rate of the substrate sample (108), and a penetration rate of the solvent (160) into the substrate sample (108) resulting from the contacting. The solvent-substrate interaction parameters (108) are determined from the set of expansion parameters.

The method allows determination of swelling rate of the substrate sample (108), and a penetration rate of the solvent (160) into the substrate sample (108). Both are useful in measuring a thermodynamic equilibrium between the solvent and the substrate. In addition, the both are useful in estimating several properties such as:
- refractive index of the substrate sample (108) and/or changes thereof during measurement of the expansion parameters;
- phase change of the substrate sample (108) during measurement of the expansion parameters;
- mechanical degradation of the substrate sample (108) and/or changes thereof during measurement of the expansion parameters;
- reaction degradation of the substrate sample (108) and/or changes thereof during measurement of the expansion parameters;
- dissolution of the substrate sample (108) during measurement of the expansion parameters.

The substrate sample (108) has a first surface (106), an opposing second surface (104). One surface (*e.g.* first surface (106)) may be smoother than the other (*e.g.* second surface (104)). The substrate sample (108) is preferably a single layer substrate.

The substrate sample is a sample (a portion) of a substrate. The substrate is a polymeric substrate.

The substrate sample has a straight side edge (102) connecting the first surface (106) and the second surface (104). Other side edges of the substrate sample may or may not be straight. In order to achieve a straight side edge (102), an edge of the substrate may be cut in a straight line. The substrate may be cut using a blade to achieve the straight side edge (102).

The substrate preferably has a thickness of from 20 to 150 micron, preferably 35 to 125 micron, even more preferably 50 to 110 micron, most preferably about 76 micron.

By 'substrate' is it meant independently a first substrate layer, a second substrate layer if present, a third substrate layer if present, or a combination thereof in a multi-layer substrate arrangement. Preferably, the substrate contains only one substrate layer.

The substrate may be soluble or dispersible in water. The substrate may be hot or cold-water soluble.

Preferred substrates exhibit good dissolution in cold water, meaning unheated distilled water. Preferably such substrates exhibit good dissolution at temperatures of 24°C, even more preferably at 10°C. By good dissolution it is meant that the substrate exhibits water-solubility of at least 50%, preferably at least 75% or even at least 95%, as measured by the method set out here after using a glass-filter with a maximum pore size of 20 microns, described above.

Preferably, the substrate has a water-solubility of at least 50%, preferably at least 75% or even at least 95%, as measured by the method set out here after using a glass-filter with a maximum pore size of 20 microns: 5 grams ± 0.1 gram of substrate material is added in a pre-weighed 3L beaker and 2L * 5ml of distilled water is added. This is stirred vigorously on a magnetic stirrer, Labline model No. 1250 or equivalent and 5 cm magnetic stirrer, set at 600 rpm, for 30 minutes at 30°C. Then, the mixture is filtered through a folded qualitative sintered-glass filter with a pore size as defined above (max. 20 micron). The water is dried off from the collected filtrate by any conventional method, and the weight of the remaining material is determined (which is the dissolved or dispersed fraction). Then, the percentage solubility or dispersability can be calculated.

The substrate may be obtained by casting, blow-moulding, extrusion or blown extrusion, as known in the art.

The substrate may take a form of a film or gel. The substrate may comprise one or more polymers. The one or more polymers may be elastomeric or non-elastomeric. The substrate may comprise superabsorbent polymer (known as SAP or AGM), acrylonitrile, butadiene styrene (ABS), polybutadiene (*e.g.* butadiene rubber, BR), polycarbonates (PC), polyethersulfones (PES), polyethylene terephthalate (PET), poly(methyl methacrylate) (PMMA) , polystyrene (PS), polysulfones (PSU), polyvinyl chloride (PVC), cellulose, or polyvinylalcohol (PVOH), or a combination or two or more of the aforementioned polymers.

The substrate may be incorporated into a product. The product is typically a manufactured product. The product preferably holds a substance (*e.g.* detergent, food product, pharmaceutical and agricultural chemical, usually solvent) sealed within the substrate by the bond. The product is preferably a soluble container or pouch containing a liquid, preferably containing a liquid detergent. The pouch is particularly used to contain a dose laundry detergent. Because the substrate is soluble, it enables release of liquid during washing. The pouch typically contains at least one lumen for holding the substance, where the lumen is defined by an upper substrate wall, a lower substrate wall, and edges which are bonded by wetting of the substrate.

The solvent may comprise water (*e.g.* tap water, de-ionised water, or distilled water), methylene, methyl ethyl ketone, tetrahydrofuran, cyclohexane, toluene, ethylene dichloride, methylene chloride, cyclohexanone, or acetone, or a combination of one or more of the aforementioned in a miscible mixture.

The solvent may be chosen according to the polymer comprised in the substrate. Examples of substrate (polymer)-solvent pairs include:
ABS - methylene, ABS - methyl ethyl ketone, ABS - tetrahydrofuran,
BR - cyclohexane, BR-toluene,
PC - ethylene dichloride, PC - methylene chloride, PC - methyl ethyl ketone,
PES - methylene chloride, PES - toluene, PET - cyclohexanone,
PMMA - cyclohexanone, PMMA - ethylene dichloride, PMMA - methylene chloride, PMMA - methyl ethyl ketone, PMMA - tetrahydrofuran,
PS - acetone, PS - ethylene dichloride, PS - methylene chloride, PS - methyl ethyl ketone, PS - toluene,
PSU - toluene, PSU - methylene chloride, PSU - methyl ethyl ketone,
PVC - acetone, PVC - cyclohexane, PVC - methyl ethyl ketone, PVC - tetrahydrofuran,
PVA - water.

To achieve the contacting of the straight side edge (102) of the substrate sample (108) with the solvent (160), the substrate sample is disposed onto a solid support. Solvent is introduced onto the solid support such that it flows towards the straight side edge (102). Preferably, only the straight side edge (102) is contacted with the solvent (160).

Preferably, the substrate sample (108) is disposed suspended or clamped in a flow cell, and the solvent (160) is introduced to the straight side edge (102) under a force of gravity or by capillary action. In a preferred example, the flow cell comprises an upper layer of transparent material and a lower layer of transparent, translucent or opaque material, and the substrate sample is suspended between the upper layer and lower layer. The layers are preferably rigid. A longitudinal channel (*e.g*. engraved channel) is provided preferably in the upper layer on the side facing the lower layer. A first end of the channel is connected to a well (hole) in the upper layer and a second end of the channel terminates adjacent to (but without overlapping with) the straight side edge (102) of the substrate sample (108). Solvent (160) introduced into the well flows along the channel under gravity, and passes from the second end of the channel to the straight side edge (102) by capillary action. The upper layer of transparent material allows passage of transmitted or reflected light for capture by the microscope.

The quantification of the solvent-substrate interaction parameters are determined from the set of expansion parameters.

By determined, it may mean that solvent-substrate interaction parameters comprises the set of expansion parameters, and the quantities of the parameters in the set expansion parameters is the same as in the solvent-substrate interaction parameter

By determined, it may mean that solvent-substrate interaction parameters comprises the set of expansion parameters, wherein one or more of the expansion parameters quantities in the set of expansion parameters have been transformed. An example of a transformation may be a scale or offset adjustment.

Swelling rate is determined from a (change in) position (*p1*) over time of the straight side edge (102) during the contacting, when viewed on the first surface (106). The position (*p1*) over time of the straight side edge (102) is determined relative to the position (*p0*) of the straight side edge (102) prior to contact with the solvent (160).

Penetration rate is determined from a (change in) position (*p2*) over time of the solvent front (162) into the substrate sample during the contacting, when viewed on the first surface (106) (**FIGs. 2** and **3**) . The position (*p2*) over time of the solvent front (162) is determined relative to the position (*p0*) of the straight side edge (102) prior to contact with the solvent (160).

The change in position is preferably determined with respect to a measurement axis (a-a') disposed perpendicular to the straight side edge (102).

As shown, for instance, in **FIG. 2**, the substrate sample (108) straight side edge (102) is brought into contact with a solvent front (162) of the solvent (160). During contacting (*e.g.* **FIG. 3**), the position of solvent front (162) changes (*p0 cf p2*) due to advancing into the substrate sample (108) and simultaneously the position of the straight side edge (102) moves (*p0 cƒ p1*) due to swelling of the substrate sample (108). A measurement axis (a-a') is indicated.

The expansion parameters may be measured optically, preferably using a light microscope. The light microscope may operate in transmission mode (Brightfield or polarized light) or in reflection mode. The microscope is disposed with an image capture device, comprising an imaging sensor for recording of images of the substrate sample (108). An example of a suitable light microscope is Zeiss Axiovert 200M.

Optical image is taken wherein at least a part of the first surface (106) is orientated to face the imaging device, in particular, the imaging sensor. Optical image is taken wherein the first surface (106) is within a focal plane of the microscope.

Optical image is taken wherein at least a part of the straight side edge (102) is within the field of view the microscope. Optical image is taken wherein at least a part of the straight side edge (102) is within a focal plane of the microscope.

The substrate sample may be disposed in any orientation with respect to the global horizon.

Because both the straight side edge (102) and the solvent front (162) can be captured in the same frame (optical image), the swelling rate of the substrate sample (108), and a penetration rate of the solvent (160) into the substrate sample (108) can be determined from a single measurement, thereby avoiding separate experiments, and savings costs and time. Moreover, both the position (*p1*) over time of the straight side edge (102), and the position (*p2*) over time of the solvent front (162) can be determined relative to the same point of reference, namely the position (*p0*) of the straight side edge (102) prior to contact with the solvent (160). This internal reference point allows more consistency and reproducibility of the expansion parameters.

A plurality of optical images is captured by the image capture device during contacting the straight side edge (102) of the substrate sample with the solvent (160).

An image dataset comprises a plurality of optical images (200, a,b,c). Each image depicts at least a portion of the first surface (106) and of the straight side edge (102) of the substrate sample. Each optical image (200, a, b, c) in the image dataset is captured at a different time point during the contacting. At least one optical image of the plurality is captured prior to contacting the straight side edge (102) with the solvent (160).

**FIGs. 4A** to **4C** exemplarily show a plurality of images (200, a to c) during contacting the straight side edge (102) of the substrate sample with the solvent (160), each image taken at a different time point (t=1 (**FIG. 4A**), t=2 (**FIG. 4B**), t=z (**FIG. 4C**)). Each image has at least:
- a first front edge (202, a,b,c) corresponding to a straight side edge (102) of the substrate sample (108), and
- a second front edge (262; a,b,c) corresponding to a solvent front (162) of the solvent (160) penetrating the substrate sample (108).

Each image (200, a to c) may further show
- an solvent part (260) corresponding to the solvent (160);
- a substrate sample part (208) corresponding to the substrate sample (108);
- a first surface part (206) corresponding to the first surface (106);

The position of the first front edge (202, a,b,c) prior to contact of the substrate sample with the solvent and the position of the second front edge (262a,b,c) prior to penetration into the substrate is shown by line *p0.* The change of position (*p1*) of the first front edge (222, a,b,c) and the change in position (*p2*) of the second front edge (262, a,b,c) may be determined relative to the position (*p0*) of the first front edge (222, a,b,c) prior to contact with the solvent.

The position (*p0*, *p1*) of the first front edge (202, a,b,c), the position (*p2*) of second front edge (262a,b,c), and may be determined for the full frame optical image (200,a,b,c), or for (only) an image portion (200',a,b,c), that is a subregion of the optical image (200,a,b,c) that contains the straight side edge (102) and solvent front (162) both before swelling has begin and until the swelling has stopped. The position and size of the image portion (200',a,b,c) remains the same for each optical image (200,a,b) in the image dataset. The image portion (200',a,b,c) is preferably rectangular (*e.g*. square or oblong) . One straight edge of image portion (200',a,b) is preferably disposed parallel to the first front edge (202, a,b,c), or disposed parallel to an average of the first front edge (202, a,b,c) in optical images (200,a,b,c) of the image dataset.

Some time after contacting, the position (*p1*) of the first front edge (202, a,b,c) changes with respect to the starting position (*p0*)*.* The change is due to swelling of the substrate sample. By measuring the change in *p1* over time, the swelling rate of the substrate sample (108) can be determined from the plurality of optical images (200, a to c) or image portions (200',a,b,c). More in particular, by measuring the change in *p1* over time relative to *p0*, the swelling rate of the substrate sample (108) can be determined from the plurality of optical images (200, a to c) or image portions (200',a,b,c).

Some time after contacting, the position (*p2*) of the second front edge (262a,b,c) changes with respect to the starting position (*p0*). The change is due to advancing penetration of the solvent (160) into the substrate sample (108). By measuring the change in *p2* over time, the penetration rate of the solvent (160) can be determined from the plurality of optical images (200, a to c) or image portions (200',a,b,c). More in particular, by measuring the change in *p2* over time relative to *p0*, the penetration rate of the solvent (160) can be determined from the plurality of optical images (200, a to c) or image portions (200',a,b,c).

The set of expansion parameters is preferably measured comprising the steps:
- generating an image dataset comprising a plurality of optical images (200, a,b,c) of at least a portion of the straight side edge (102), each optical image (200, a, b, c) captured at a different time point during the contacting; and
- identifying in each optical image (200, a, b, c) or image portion (200',a,b,c) thereof a first front edge (202, a,b,c) corresponding to a straight side edge (102) of the substrate sample (108), and a second front edge (262a,b,c) corresponding to a solvent front (162) of the solvent (160) penetrating the substrate sample (108); and
wherein the swelling rate is determined from a change in position (*p1*) of the first front edge (202, a,b,c) over the different timepoints, and the penetration rate is determined from a change in position (*p2*) of the second front edge (262a,b,c) over the different timepoints.

Preferably, images are collected for 8 to 20 seconds, preferably 8 to 14 seconds after initial contact of straight side edge (102) with the solvent (160).

The first front edge (202, a,b,c) and second front edge (262a,b,c) may be automatically identified in each optical image (200, a,b,c) or in each image portion (200',a,b,c) by enhancing the optical image (200, a,b,c) or image portion (200',a,b,c) to emphasise the first front edge (202, a,b,c) and the second front edge (262a,b,c). Thereby, the swelling rate and penetration rate may be automatically measured. The image (200, a,b,c) so enhanced is an enhanced image (300, a,b). The image portion (200',a,b) so enhanced is an enhanced image portion (300',a,b). Examples of enhanced images (300, a,b) are shown in **FIGs. 6A** and **6B**, which are generated from the respective optical images (200, a,b) and optical image portions (200, a,b) shown in **FIGs. 5A** and **B** at times t=1 and t=2 respectively. Examples of enhanced image portions (300', a,b) are shown in **FIGs. 9A** and **9B**, which are generated from the respective optical images (200, a,b) and optical image portions (200, a,b) shown in **FIGs. 8A** and **8B** at times t=1 and t=2 respectively.

The first front edge (202, a,b) in the optical image (200, a,b) or image portion (200',a,b) corresponds to an enhanced first region (302, a,b) in the enhanced image (300, a,b) or enhanced image portion (300',a,b). The second front edge (262, a,b) in an optical image (200, a,b) image portion (200',a,b) corresponds to an enhanced second region (362,a,b) in the enhanced image (300, a,b) or the enhanced image portion (300',a,b).

Preferably, the enhanced first region (302, a,b,c) has a grey intensity that is different compared with regions flanking the enhanced first region (302, a,b,c). Preferably, the enhanced first region (302, a,b,c) has a grey intensity that is higher (lighter) compared with regions flanking the enhanced first region (302, a,b,c). Where, on a numeric scale of grey intensity, a darker shade has a lower value than a lighter shade, preferably the enhanced first region (302, a,b,c) has a grey intensity having a greater value compared with regions flanking the enhanced first region (302, a,b,c). Where, on a numeric scale, a grey intensity of 0 is black and of 255 is white, preferably the enhanced first region (302, a,b,c) has a grey intensity having a greater value compared with regions flanking the enhanced first region (302, a,b,c), preferably at least 3% to 10% greater.

Preferably, the enhanced second region (362, a,b) has a grey intensity that is different compared with regions flanking the enhanced second region (362, a,b). Preferably, the enhanced second region (362, a,b) has a grey intensity that is higher (lighter) compared with regions flanking the enhanced second region (362, a,b). Where, on a numeric scale of grey intensity, a darker shade has a lower value than a lighter shade, preferably the enhanced second region (362, a,b) has a grey intensity having a greater value compared with regions flanking the enhanced second region (362, a,b). Where on a numeric scale a grey intensity of 0 is black and of 255 is white, preferably the enhanced second region (362, a,b) has a grey intensity having a greater value compared with regions flanking the enhanced second region (362, a,b), preferably at least 3% to 10% greater.

The enhanced first region (302, a,b,c) and the enhanced second region (362, a,b,c) have the highest grey intensities compared with other regions (e.g. regions between them, or to either side of them). Having the highest intensities allows them to be automatically identified.

Preferably, the enhancing comprises the steps:
- for each optical image (200, a,b,c ) in the image dataset or image portion (200', a, b, c) thereof in the image dataset:
   - with the first front edge (202, a,b,c) aligned parallel to a y-axis, and an x-axis being perpendicular to the y-axis;
   - applying a first filter to the optical image (200, a,b) or image portion (200',a,b), comprising replacing each pixel of each column (C1 to C4) of pixels parallel to the y-axis into an average of grey intensity for that column, a column being parallel to the y-axis;
   - optionally applying (consecutively) one or more edge-enhancing filters to each previously-filtered image;
   - thereby obtaining an enhanced image (300, a,b) or enhanced image portion (300',a,b).

An example of an arrangement of pixels of the optical image (200) or of the image portion thereof (200) is shown in **FIG. 11**, wherein each column (C1 to C4) of pixels grey intensities is parallel to an average of direction of the first front edge (202, a,b,c). In **FIG. 12**, the pixels in each column (C1 to C4, FIG. 11) of optical image (200) or of the image portion thereof (200) have each been replaced with a value that is an average of pixels for each column (C1' to C4').

The edge-enhancing filter may be:
- a Sobel filter applied along the x-axis (1st derivative of grey intensity); or
- a Laplace filter applied along the x-axis.

Preferably after applying the first filter, the Sobel filter is applied, followed by the Laplace filter.

The Sobel filter is known in the art. See for instance Kanopoulos, N., Vasanthavada, N., & Baker, R. L. (1988) Design of an image edge detection filter using the Sobel operator, IEEE Journal of Solid-State Circuits, 23(2), 358-367. The filter is applied along the x-axis. The kernel size depends on the size of the optical image (200, a,b,c) or in each image portion (200',a,b,c). As a guidance, values between 20 and 40 may be used. An example for software suitable for applying the Sobel filter includes Open CV (opencv.org).

The Laplace filter is known in the art. See for instance Dhar, R., Gupta, R., and Baishnab, K.L. (2014) An Analysis of CANNY and LAPLACIAN of GAUSSIAN Image Filters in Regard to Evaluating Retinal Image, DOI: 10. 1 109/ICGCCEE.2014.6922270. The filter is applied along the x-axis. The kernel size depends on the size of the optical image (200, a,b,c) or in each image portion (200',a,b,c). As a guidance, values between 20 and 40 may be used. An example for software suitable for applying the Laplace filter includes Open CV (opencv.org).

The enhanced first region (302, a,b) and the enhanced second region (362, a,b) are bands both aligned with the y-axis.

The enhanced first region (302, a,b) corresponds to the first front edge (202, a,b,c) of the optical image (200,a,b) or image portion (200',a,b), that corresponds to the straight side edge (102) of the substrate sample (108). The position (*p0*, *p1*) of the enhanced first region (302,a, b) corresponds to the position (*p0*, *p1*) of the first front edge (202, a,b,c). By measuring the change in *p1* over time (relative to *p0*), the swelling rate of the substrate sample (108) can be determined from the plurality of images (200, a to c) or image portions (200',a,b).

The enhanced second region (362,a, b) corresponds to the second front edge (262a,b,c) of the optical image (200,a,b) or image portion (200',a,b), that corresponds to the solvent front (162) of the solvent (160) penetrating the substrate sample (108). The position (*p2*) of the enhanced second region (362,a, b) corresponds to the position (*p2*) of the second front edge (262, a,b,c). By measuring the change in *p2* over time (relative to *p0*), the penetration rate of the solvent can be determined from the plurality of images (200, a to c) or image portions (200',a,b).

The image portion (200',a,b) may be rectangular (*e.g*. square or oblong) and rotated relative to the corresponding optical image (200, a,b) such that one straight edge of the rectangle is parallel with the first front edge (202, a,b,c). In other words, one straight edge of the rectangle is aligned parallel with the aforementioned y-axis. The enhancing may be performed on the image portion (200',a,b).

The one straight edge of the rectangular image portion (200',a,b) may aligned parallel with the first front edge (202, a,b,c) by a protocol comprising the steps:
- for each optical image (200, a,b,c) in the image dataset:
   - applying a Gaussian smoothing filter;
   - subsequently applying a Laplace filter (highlight edges);
   - subsequently thresholding the image with Otsu's method;
   - subsequently identify the strongest edge lines using a Hough transform;
   - subsequently fitting *a* first line to the edge identified by the Hough transform;
   - subsequently generate a second line perpendicular to the first line;
   - rotating rectangular image portion (200',a,b) such that one straight edge of the image portion (200') that it is parallel to an average rotation of the second line of the optical images (200, a,b,c) in the image dataset.

The Gaussian smoothing filter is known in the art. See for instance en.wikipedia.org/wiki/Gaussian_filter. As a guidance, an aperture of between 20 and 40 may be used. An example for software suitable for applying the Gaussian smoothing filter includes Open CV (opencv.org).

The Laplace filter is known in the art, as mentioned elsewhere herein. The filter is applied along the x-axis. The kernel size depends on the size of the optical image (200, a,b,c) or in each image portion (200',a,b,c). As a guidance, values between 20 and 40 may be used.

Otsu's method is known in the art. See for example, Nobuyuki Otsu, A threshold selection method from gray-level histograms (1979) IEEE Trans. Sys. Man. Cyber. 9 (1): 62-66. doi:10.1109/TSMC.1979.4310076. An example for software suitable for applying Otsu's method includes Open CV (opencv.org).

Hough transform is known in the art. See for example, U.S. Patent 3,069,654, en.wikipedia.org/wiki/Hough_transform. Purely as guidance, the following parameters were set resolution=1, angleresolution=1radian, threshold=20, minLineWidth=30% of image width, gap_between_lines=10. An example for software suitable for applying the Hough transform includes Open CV (opencv.org).

The position and rotation of the rectangular image portion (200',a,b) is the same for every optical image (200, a,b,c) in the image dataset.

The enhanced first region (302,a, b) and enhanced second region (362,a, b)) may be automatically identified from the enhanced image (300,a, b) by generating an intensity profile (400,a,b) from the enhanced image (300,a, b) or enhanced image portions (300', a,b).

The intensity profile (400,a, b) indicates along one axis (*e.g*. y-axis) pixel intensity of the enhanced image (302,a,b) or enhanced image portion (300', a,b) as an amplitude, and along another axis (*e.g.* x-axis) position along the x-axis of the enhanced image (302,a,b) or enhanced image portion (300', a,b). Since the pixels have the same column intensity, the x-axis of the enhanced image (302,a,b) or enhanced image portion (300', a,b) can be at any y-axis position. A first peak (402,a,b) in the intensity profile (400,a,b) corresponds to the enhanced first region (302,a, b). A second peak (462,a,b) in the intensity profile (400,a,b) corresponds to enhanced second region (362,a, b). Examples of intensity profiles (400,a,b) are shown in **FIGs. 7A** and **7B**, which are generated from the respective enhanced image (302,a,b) shown in **FIGs. 6A** and **6B** at times t=1 and t=2 respectively. Examples of intensity profiles (400,a,b) are shown in **FIGs. 10A** and **10B**, which are generated from the respective enhanced image portions (300', a,b) shown in **FIGs. 9A** and **9B** at times t=1 and t=2 respectively.

The first peak (402,a,b) and the second peak (462,a,b) have the highest maximum amplitudes compared with other regions (*e.g.* regions between them or to the side of them). Having the maximum amplitudes allows them to be automatically identified.

The first peak (402,a,b) in the intensity profile (400,a,b) corresponds to the enhanced first region (302,a, b), that corresponds to the first front edge (202, a,b,c), that corresponds to the straight side edge (102) of the substrate sample (108). The position (*p0*, *p1*) of the first peak (402,a,b) corresponds to the position (*p0*, *p1*) of the enhanced first region (302,a, b), that corresponds to the position (*p0*, *p1*) of the first front edge (202, a,b,c). By measuring the change in *p1* over time (relative to *p0*), the swelling rate of the substrate sample (108) can be determined from the plurality of images (200, a to c).

The second peak (462,a,b) in the intensity profile (400,a,b) corresponds to enhanced second region (362,a, b), that corresponds to the second front edge (262a,b,c), that corresponds to the solvent front (162) of the solvent (160) penetrating the substrate sample (108). The position (*p2*) of the second peak (462,a,b) corresponds to the position (*p2*) of the enhanced second region (362,a, b) that corresponds to the position (*p2*) of the second front edge (262, a,b,c). By measuring the change in *p2* (relative to *p0*) over time, the penetration rate of the solvent can be determined from the plurality of images (200, a to c).

Preferably, the first peak (402,a,b) has a maximum amplitude that is different compared with regions flanking the first peak (402,a,b). Where, on a numeric scale, a grey intensity of 0 is black and of 255 is white, preferably the first peak (402,a,b) has a maximum amplitude that has a greater value compared with regions flanking the first peak (402,a,b), preferably at least 3% to 10% greater.

Preferably, the second peak (462,a,b) has a maximum amplitude that is different compared with regions flanking the first peak (402,a,b). Where, on a numeric scale, a grey intensity of 0 is black and of 255 is white, preferably the second peak (462,a,b) has a maximum amplitude that has a greater value compared with regions flanking the second peak (462,a,b), preferably at least 3% to 10% greater.

Optionally one or more noise-reducing filters may be applied to the enhanced image (300,a, b) (or image portion (300',a, b) thereof) and/or to the intensity profile (400,a, b). The noise-reducing filter may be:
- a cut-off filter, to remove or diminish parts of the enhanced image (300,a, b) (or image portion (300',a, b) thereof) and/or of the intensity profile (400,a, b) which are below an intensity or amplitude threshold value.
- a stationary-selective-filter to remove or diminish parts of the enhanced image (300,a, b) (or image portion (300',a, b) thereof) and/or of the intensity profile (400,a, b) that do not change in position over the x-axis over a majority of the different time points.
- a lifespan-selective-filter to remove or diminish grey portions of the intensity profile (400,a, b) that are short-lived.

The cut-off filter is known in the art, and discards the edges (enhanced first region (302, a,b) or enhanced second region (362,a, b)) whose peak value are below the threshold. Alternatively, edges or peaks above the threshold are used to determine the position (p1) of the straight side edge (102) and position (p2) of the solvent front (162).

The stationary-selective-filter analyses all the enhanced images (300,a, b) (or image portions (300',a, b) thereof). (200',a,b) or intensity profiles (400,a, b) determined from the image dataset. The stationary-selective-filter analyses the position history of each single edge (enhanced first region (302, a,b) or enhanced second region (362,a, b)) or peak (in the intensity profiles (400,a, b)). A linear profile is fitted to a curve of each edge position or peak position over time. If the slope of this linear line is below a threshold indicated by the user (*e.g.* 4 pixels/sec), the edges or peaks are discarded. Edges or peaks that change position (slope is above a threshold) are used to determine the straight side edge (102) and the solvent front (162) in the .

The lifespan-selective-filter retains only those edges (within the enhanced images (300,a, b)) or peaks (within the intensity profiles (400,a, b)) that appear in a minimum number of consecutive frames. As a guidance, with a frame capture rate of 20 to 30 frames per second, edges or peaks appearing in at least 80 to 100 consecutive frames are retained.

The swelling rate is determined from the position (*p1*) of the straight side edge (102) of the substrate sample (108) over time during contacting with the solvent. The position (*p1*) of the straight side edge (102) may be determined relative to the position (*p0*) of straight side edge (102) prior to contact with the solvent.

The straight side edge (102) prior to and during contact with the solvent may be mutually parallel. A distance between the position (p1) of the straight side edge (102) of the substrate sample (108) during contact with the solvent and the position (*p0*) of the straight side edge (102) prior to contact with the solvent may be considered along an axis perpendicular to the straight side edge (102) before or during contact with the solvent.

The swelling rate may be determined from a time averaged window of the position (*p1*) of the straight side edge (102) over the different timepoints.

Swelling rate may be determined from the position (*p1*) at the different time points of the first front edge (202,a,b,c) each optical image (200, a,b,c) or image portion (200', a,b,c) in the image dataset. The position of the first front edge (202,a,b,c) (*p1*) may be determined relative to the position (*p0*) of first front edge (202,a,b,c) prior to contact with the solvent.

The first front edge (202,a,b,c) prior to and during contact with the solvent may be mutually parallel. A distance between the position (p1) of the first front edge (202,a,b,c) during contact with the solvent and the position (*p0*) of the first front edge (202,a,b,c) prior to contact with the solvent may be considered along an axis perpendicular to the first front edge (202,a,b,c) before or during contact with the solvent.

The swelling rate may be determined from a time averaged window of the position (*p1*) of the first front edge (202,a,b,c) over the different timepoints.

Swelling rate may be determined from the position (*p1*) at the different time points of the enhanced first region (302,a, b) in the enhanced image (300,a,b) or enhanced image portion (300', a,b,c). The position of the enhanced first region (302,a, b) (*p1*) may be determined relative to the position (*p0*) of enhanced first region (302,a, b) prior to contact with the solvent.

The enhanced first region (302,a, b) prior to and during contact with the solvent are mutually parallel. A distance between the position (p1) of the enhanced first region (302,a, b) during contact with the solvent and the position (*p0*) of the enhanced first region (302,a, b) prior to contact with the solvent may be considered along an axis perpendicular to the enhanced first region (302,a, b) before or during contact with the solvent.

The swelling rate may be determined from a time averaged window of the position (*p1*) of the enhanced first region (302,a, b) over the different timepoints.

Swelling rate may be determined from the position (p1) at the different time points of the first peak (402,a,b) in the intensity profile (400,a,b). The position of the first peak (402,a,b) (*p1*) may be determined relative to the position (*p0*) of first peak (402,a,b) prior to contact with the solvent.

The swelling rate may be determined from a time averaged window of the position (*p1*) of the first peak (402,a,b) over the different timepoints.

Swelling rate may be non-scalar e.g. may be represented by a one or more parameters, by function, by a graph etc, as an evolution in the position (*p1*) over time. Swelling rate may be scalar and represented by a value. The swelling rate may be represented by a set of parameters, obtaining by fitting a first function, for instance, a first exponential function, to a curve corresponding to the position (*p1*) of the first front edge (222) at the different time points,

One example of the first function is an exponential curve Aₛ+Bₛ^{∗}exp(-Cₛ^{∗}t). Aₛ, Bₛ and Cₛ are variables that are adjusted until the function fits the curve. The parameter Cₛ corresponds to the swelling rate.

Another example of the function is a model based on Mao *et al* (Mao et al «Hydration and swelling of dry polymers for wet adhesion»). The output is a more accurate estimation of the penetration depth and swelling rate.

The penetration rate is determined from the position (*p2*) of the solvent front (162) of the solvent (160) penetrating the substrate sample (108) over time during contacting with the solvent. The position of the solvent front (162) may be determined relative to the position (*p0*) of straight side edge (102) prior to contact with the solvent.

The solvent front (162) prior to and during contact with the solvent may be mutually parallel. A distance between the position (p2) of the solvent front (162) during contact with the solvent and the position (*p0*) of the straight side edge (102) prior to contact with the solvent may be considered along an axis (a-a') perpendicular to the straight side edge (102) before or during contact with the solvent.

The penetration rate may be determined from a time averaged window of the position (*p1*) (relative to *p0*) of the solvent front (162) over the different timepoints.

Penetration rate may be determined from the position (*p2*) at the different time points of the second front edge (262,a,b,c) each optical image (200, a,b,c) or image portion (200', a,b,c) in the image dataset. The position (*p2*) of the second front edge (262,a,b,c) may be determined relative to the position (*p0*) of first front edge (202,a,b,c) prior to contact with the solvent.

The second front edge (262,a,b,c) prior to and during contact with the solvent may be mutually parallel. A distance between the position (*p2*) of the second front edge (262,a,b,c) during contact with the solvent and the position (*p0*) of the first front edge (202,a,b,c) prior to contact with the solvent may be considered along an axis (a-a') perpendicular to the first front edge (202,a,b,c) before or during contact with the solvent.

The penetration rate may be determined from a time averaged window of the position (*p2*) (relative to *p0*) of the second front edge (262,a,b,c) over the different timepoints.

Penetration rate may be determined from the position (*p2*) at the different time points of the enhanced second region (362,a, b) in the enhanced image (300,a,b) or enhanced image portion (300',a,b). The position of the enhanced second region (362,a, b) (*p2*) may be determined relative to the position (*p0*) of enhanced first region (302,a, b) prior to contact with the solvent.

The enhanced second region (362,a, b) prior to and during contact with the solvent are mutually parallel. A distance between the position (*p2*) of the enhanced second region (362,a, b) during contact with the solvent and the position (*p0*) of the enhanced first region (302,a, b) prior to contact with the solvent may be considered along an axis (a-a') perpendicular to the enhanced first region (302,a, b) before or during contact with the solvent.

The penetration rate may be determined from a time averaged window of the position (*p2*) (relative to *p0*) of the enhanced second region (362,a, b) over the different timepoints.

Penetration rate may be determined from the position (*p2*) at the different time points of the second peak (462,a,b) in the intensity profile (400,a,b). The position of the second peak (462,a,b) (*p2*) may be determined relative to the position (*p0*) of first peak (402,a,b) prior to contact with the solvent.

The penetration rate may be determined from a time averaged window of the position (*p2*) (relative *to p0)* of the second peak (462,a,b) over the different timepoints.

Penetration rate may be non-scalar *e.g.* may be represented by a one or more parameters, by function, by a graph etc, as an evolution in the position (*p2*) over time. Penetration rate may be scalar and represented by a value. The penetration rate may be represented by a set of parameters, obtaining by fitting a second function, for instance, a second exponential function, to the position (*p2*) of the second front edge (262) at the different time points.

One example of the second function is an exponential curve Aₚ+Bₚ^{∗}exp(-Cₚ^{∗}t). Aₚ, Bₚ and Cₚ are variables that are adjusted until the function fits the curve. The parameter Cₚ corresponds to the penetration rate.

Another example of the function is a model based on Mao *et al* (Mao et al, Hydration and swelling of dry polymers for wet adhesion, Journal of the Mechanics and Physics of Solids, Volume 137, April 2020, 103863)

Provided herein is a computer-implemented method for quantification of solvent-substrate interaction parameters of a solvent (160) and a substrate sample (108) interaction using a method as described herein.

Further provided herein is a system comprising a processor adapted to perform the computer-implemented method.

The system may further comprise:
- a light microscope disposed with an image capture device configured for acquisition of the image dataset.

Further provided herein is a computer program [product] comprising instructions which, when the program is executed by a computer, cause the computer to carry out the computer-implemented method.

Further provided herein is a computer-readable [storage] medium comprising instructions which, when executed by a computer, cause the computer to carry out [the steps of] the computer-implemented method.

Provided herein is a computer-implemented method for a quantification of solvent-substrate interaction parameters of a solvent (160) and a substrate sample (108) interaction , comprising:
- receiving an image dataset comprising a plurality of optical images (200, a,b,c) of at least a portion of a straight side edge (102) of the substrate sample (108), each optical image (200, a,b,c) captured at a different time point during contacting the straight side edge (102) of the substrate sample (108) with an solvent (160),
- identifying in each optical image (200, a,b,c) or an image portion (200', a,b,c) thereof, a first front edge (202, a,b,c) corresponding to the straight side edge (102), and a second front edge (262a,b,c) corresponding to a solvent front (162) of the solvent (160) penetrating the substrate sample (108); and
- determining a set of expansion parameters comprising:
   - a swelling rate of the substrate sample (108), and
   - a penetration rate of the solvent (160) into the substrate sample (108),
      wherein the swelling rate is determined from a change in position (*p1*) of the first front edge (202, a,b,c) over the different timepoints, and the penetration rate is determined from a change in position (*p2*) of the second front edge (262a,b,c) over the different timepoints,
- determining from the expansion parameters, the quantification of the solvent-substrate interaction parameters.

Any of the aforementioned features described herein may be applied to the computer-implemented method.

Further provided herein is a system comprising a processor adapted to perform the computer-implemented method. The processor may be comprised in a remote server (cloud)

Further provided herein is a computer program [product] comprising instructions which, when the program is executed by a computer, cause the computer to carry out the computer-implemented method.

Further provided herein is a computer-readable [storage] medium comprising instructions which, when executed by a computer, cause the computer to carry out [the steps of] the computer-implemented method.

The computer implemented method may output the result in a report.

Provided herein is a computer-implemented method for a quantification of solvent-substrate interaction parameters of a solvent (160) and a substrate sample (108) interaction , comprising:
- sending, to a remote processor, an image dataset comprising a plurality of optical images (200, a,b,c) of at least a portion of a straight side edge (102) of the substrate sample (108), each optical image (200, a,b,c) captured at a different time point during contacting the straight side edge (102) of the substrate sample (108) with an solvent (160);
- receiving from the remote processor, the quantification of solvent-substrate interaction parameters, wherein the quantification of solvent-substrate interaction parameters has been determined by:
   - identifying in each optical image (200, a,b,c) a first front edge (202, a,b,c) corresponding to the straight side edge (102), and a second front edge (262a,b,c) corresponding to a solvent front (162) of the solvent (160) penetrating the substrate sample (108); and
   - determining a set of expansion parameters comprising:
      - a swelling rate of the substrate sample (108), and
      - a penetration rate of the solvent (160) into the substrate sample (108),
         wherein the swelling rate is determined from a change in position (*p1*) of the first front edge (202, a,b,c) over the different timepoints, and the penetration rate is determined from a change in position (*p2*) of the second front edge (262a,b,c) over the different timepoints,
   - determining from the expansion parameters, the quantification of the solvent-substrate interaction parameters.

Further provided is a system comprising a processor adapted to perform the computer implemented method. A part of the processor may be comprised in a remote server (cloud).

The system may further comprise:
- a light microscope disposed with an image capture device configured for acquisition of the image dataset.

Further provided is a computer program [product] comprising instructions which, when the program is executed by a computer, cause the computer to carry out [the steps of] the computer implemented method.

Further provided is a computer-readable [storage] medium comprising instructions which, when executed by a computer, cause the computer to carry out [the steps of] the computer implemented method.

The computer implemented method may output the result in a report.

The computer implemented method, system and computer program allow the determination to be performed remotely, based on an image dataset. A local user may perform the contacting of the straight side edge (102) of the substrate sample with the solvent (160), and capturing of optical images. A central (*e.g.* cloud-based) server may receive the dataset and determine the quantification of solvent-substrate interaction parameters from the image dataset. A central server allows for the method to be performed on local devices not having adequate image processing capability, and avoid the need for locally updating software to account for changes in the determining steps. It allows for remote prediction of bonding strength at a location of manufacture of the substrate that is remote from location of product manufacture; the quality of the substrate can be assessed prior to shipping to the location of product manufacture, and prior to usage in the product manufacturing.

The method may be performed using a standard computer system such as an Intel Architecture IA-32 based computer system 2, and implemented as programming instructions of one or more software modules stored on non-volatile (e.g., hard disk or solid-state drive) storage associated with the corresponding computer system. However, it will be apparent that at least some of the steps of any of the described processes could alternatively be implemented, either in part or in its entirety, as one or more dedicated hardware components, such as gate configuration data for one or more field programmable gate arrays (FPGAs), or as application-specific integrated circuits (ASICs), for example.

The method or system may produce an output that is:
- displayed on a screen, or
- saved to a file.

### Example

Sample of substrate containing polyvinylalcohol was cut with a guillotine to form a straight side edge. The substrate sample was mounted within a flow cell as described herein and solvent applied to the straight side edge. A plurality of optical images was recorded, at least one optical image before contacting of the substrate sample with the solvent. Images were taken for a standard duration of 15secs. The plurality of image were stored in an image dataset.

**FIG. 13** is a microscope optical image (200,d) of the substrate sample (208) and solvent (260) at time t=0, *i.e.* just prior to contact. The straight side edge (102,d), line (*p0*), and a rectangular image portion (200',d) are marked. **FIG. 14** is a microscope optical image (200,f) of the same substrate sample (208) and solvent (260) after contacting for 12 seconds (t=12 s). The straight side edge (102,f) corresponding line (*p0*), solvent front (262,f), and rectangular image portion (200',f) are marked.

The rectangular image portion was determined for each image in the dataset. The position and rotation of the rectangular image portion was the same for each image. The position and rotation were determined by applying, for each image in the dataset, a Gaussian smoothing filter, subsequently a Laplace filter (highlight edges), subsequently thresholding the image with Otsu method and subsequently identifying the strongest edge line using a Hough transform. A first line was fit to this line. One y-axis straight edge of the rectangular image portion was disposed parallel to an average rotation of the first line across all the images in the dataset. The position was determined such that rectangular image portion contained both the straight side edge and solvent front both before swelling had begum and until the swelling has stopped. **FIG. 15** shows one of the optical images (200,e) of the substrate sample (208) in the dataset taken at t=0s *i.e.* just prior to contacting, with the determined image portion (200',e) indicated.

Each rectangular image portion, having a y-axis and an x-axis perpendicular thereto was enhanced to form an enhanced portion by applying a first filter to the image portion. The first filter comprised replacing each pixel of each column of pixels parallel to the image portion y-axis into an average of grey intensity for that column, subsequently applying a Sobel filter along the x-axis, subsequently applying a Laplace filter along the x-axis.

**FIG. 16** (t=0s) shows the rectangular image portion (200',e) of FIG. 15, and the enhanced portion (300',e) generated from the image portion (200',e), after averaging each pixel column (310,e) and applying edge-detection filters (Sobel filter along the x-axis (result in 312,e), Laplace filter along the X axis (result in 300',e)) resulting in the enhanced image portion (300',e).

**FIG. 17** (t=5s) shows the rectangular image portion (200',f) generated from an image in the dataset captured five seconds after the image of **FIG. 15** / **FIG. 16**, and the enhanced portion (300',f) generated from the image portion (200',f), after averaging each pixel column (310,f) and applying edge-detection filters (Sobel filter along the x-axis (result in 312,f), Laplace filter along the X axis (result in 300',f)) resulting in the enhanced image portion (300',e).

An intensity profile was generated from each enhanced image portion. To generate the intensity profile, a plot was created wherein the y-axis corresponded to pixel intensity and x-axis corresponded to position along the x-axis of the enhanced image portion. Noise reducing filters were applied: one filter being a cut-off filter, to remove or diminish portions of the intensity profile below a threshold value, another filter being a stationary-selective-filter to remove or diminish portions of the intensity profile that do not change in position over a majority of the different time points, and a lifespan-selective-filter to remove or diminish of portions of intensity profile that are short-lived. **FIG. 16** shows the intensity profile (400,e) generated from the enhanced portion (300',e) at t=0s. **FIG. 17** shows the intensity profile (400,f) generated from the enhanced portion (300',f) some seconds later (t=5s).

The resulting intensity profile contained two main peaks of intensity (highest amplitude), the first peak corresponded to the position *(p0, p1*) of the straight side edge, and the second peak corresponded to the position (*p2*) of the solvent front. From the intensity profiles, a data set of position parameters was obtained corresponding to *p1* and *p2* relative to *p0* across the time points. **FIG. 18** shows a plot of positions (*p0*, *p1*) of the straight side edge, and of positions (*p2*) of the solvent front as a function of time

A mathematic function was fitted to each of the curves of *p1* (relative to *p0*) and *p2* (relative to *p0*) over time. The mathematic function was the exponential curve A+B*exp(-C*t). The value of C for the curve of *p1* corresponds to the swelling rate, and the value of C for the curve of *p2* corresponds to the penetration rate.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A method for a quantification of solvent-substrate interaction parameters of a solvent (160) and a substrate sample (108) interaction, the substrate sample (108) having a first surface (106), an opposing second surface (104) and a straight side edge (102) connecting the first surface (106) and the second surface (104), comprising:
- contacting the straight side edge (102) of the substrate sample (108) with a solvent (160),
- measuring, from the substrate sample (108), a set of solvent expansion parameters comprising:
- a swelling rate of the substrate sample (108), and
- a penetration rate of the solvent (160) into the substrate sample (108) resulting from the contacting,
wherein the set of solvent expansion parameters is determined by:
- generating an image dataset comprising a plurality of optical images (200, a,b,c) of at least a portion of the straight side edge (102), each optical image (200, a, b) captured at a different time point during the contacting;
- identifying in each optical image (200, a,b,c) or from an image portion thereof (200', a,b,c) a first front edge (202, a,b,c) corresponding to the straight side edge (102), and a second front edge (262, a,b,c) corresponding to a front edge (162) of the solvent (160) penetrating the substrate sample (108); and
- the swelling rate is determined from a position (p1) of the first front edge (202, a,b,c) over the different timepoints, and the penetration rate is determined from a position (p2) of the second front edge (262a,b,c) over the different timepoints,
wherein the solvent-substrate interaction parameters are determined from the set of solvent expansion parameters.

2. The method according to claim 1, wherein the first (202, a,b) and second (262, a,b) front-edges are identified comprises the steps:
- for each optical image (200, a,b) in the image dataset, or image portion thereof (200', a,b):
- with the first front edge (202, a,b,c) aligned parallel to a y-axis, and an x-axis being perpendicular to the y-axis;
- applying a first filter to the optical image (200, a,b) or image portion (200',a,b), comprising replacing (C1' to C4') each pixel of each column (C1 to C4) of pixels parallel to the y-axis into an average of grey intensity for that column;
- optionally applying one or more edge-enhancing filters to each previously-filtered image;
- thereby obtaining an enhanced image (300, a,b) or enhanced image portion (300',a,b),
- identifying from the enhanced image (300, a,b) or enhanced image portion (300',a,b) thereof, an enhanced first region (302, a,b) and enhanced second region (362,a,b), the enhanced first (302, a,b) and second (362, a,b,c) regions have the highest grey intensities compared with other regions, wherein the enhanced first region (302, a,b) corresponds to the first front edge (202, a,b) and the enhanced second region (362,a,b) corresponds to the second front edge (262, a,b).

3. The method according to claim 2, wherein the identifying from enhanced image (300, a,b) the first front edge (222,a, b) and second front edge (262,a, b)) further comprises the steps:
- for each enhanced image (300, a,b) or enhanced image portion (300', a,b)
- determining an intensity profile (400,a, b) along the x-axis of the enhanced image (302,a,b),
wherein intensity profile (400,a, b) indicates along one axis pixel intensity of the enhanced image (302,a,b) or enhanced image portion (300', a,b) as an amplitude, and along another axis position along the x-axis of the enhanced image (302,a,b) or enhanced image portion (300', a,b),
- optionally applying one or more noise-reducing filters to the intensity profile (400,a, b)
- identifying in the intensity profile (400a, b), optionally noise-filtered, a first peak (402,a,b) and a second peak (462,a,b) have the highest maximum amplitudes compared with other regions, wherein the first peak (402,a,b) corresponds to the first front edge (202, a,b) and the second peak (462,a,b) corresponds to the second front edge (262a,b,c).

4. The method according to any one of the previous claims, wherein
- the swelling rate is represented as a first exponential function fitted to the position (*p1*) of the first front edge (202, a,b,c) over the different timepoints,
- the penetration rate is represented as a second exponential function fitted to the position (*p2*) of the second front edge (262, a,b,c) over the different timepoints.

5. The method according to any one of the previous claims, wherein the substrate sample (108) comprises superabsorbent polymer (SAP or AGM), acrylonitrile, butadiene styrene (ABS), polybutadiene (*e.g*. butadiene rubber, BR), polycarbonates (PC), polyethersulfones (PES), polyethylene terephthalate (PET), poly(methyl methacrylate) (PMMA), polystyrene (PS), polysulfones (PSU), polyvinyl chloride (PVC), cellulose, or polyvinylalcohol (PVOH), or a combination or two or more of the aforementioned polymers.

6. A computer-implemented method for a quantification of solvent-substrate interaction parameters of a solvent (160) and a substrate sample (108) interaction, the method comprising:
- receiving an image dataset comprising a plurality of optical images (200, a,b,c) of at least a portion of a straight side edge (102) of the substrate sample (108), each optical image (200, a,b,c) captured at a different time point during contacting the straight side edge (102) of the substrate sample (108) with an solvent (160),
- identifying in each optical image (200, a,b,c) or an image portion (200', a,b,c) thereof a first front edge (202, a,b,c) corresponding to the straight side edge (102), and a second front edge (262a,b,c) corresponding to a solvent front (162) of the solvent (160) penetrating the substrate sample (108); and
- determining a set of expansion parameters comprising:
- a swelling rate of the substrate sample, and
- a penetration rate of the solvent (160) into the substrate sample (108),
wherein the swelling rate is determined from a change in position (*p1*) of the first front edge (202, a,b,c) over the different timepoints, and the penetration rate is determined from a change in position (*p2*) of the second front edge (262a,b,c) over the different timepoints,
- determining from the set of solvent expansion parameters the solvent-substrate interaction parameters.

7. The computer implemented method according to claim 6, incorporating the subject matter of any of claims 2 to 5.

8. A system comprising a processor adapted to perform the computer-implemented method of claim 6 or 7.

9. A computer-implemented method for determining a quantification of solvent-substrate interaction parameters of a solvent (160) and a substrate sample (108) interaction, the method comprising:
- sending, to a remote processor, an image dataset comprising a plurality of optical images (200, a,b,c) of at least a portion of a straight side edge (102) of the substrate sample (108), each optical image (200, a,b,c) captured at a different time point during contacting the straight side edge (102) of the substrate sample (108) with an solvent (160);
- receiving from the remote processor, the quantification of solvent-substrate interaction parameters, wherein the quantification of solvent-substrate interaction parameters has been determined by:
- identifying in each optical image (200, a,b,c) a first front edge (202, a,b,c) corresponding to the straight side edge (102), and a second front edge (262a,b,c) corresponding to a solvent front (162) of the solvent (160) penetrating the substrate sample (108); and
- determining a set of expansion parameters comprising:
- a swelling rate of the substrate sample (108), and
- a penetration rate of the solvent (160) into the substrate sample (108),
wherein the swelling rate is determined from a change in position (*p1*) of the first front edge (202, a,b,c) over the different timepoints, and the penetration rate is determined from a change in position (*p2*) of the second front edge (262a,b,c) over the different timepoints,
- determining from the expansion parameters, the quantification of solvent-substrate interaction parameters.

10. The computer implemented method according to claim 9, incorporating the subject matter of any of claims 2 to 5.

11. A system comprising a processor adapted to perform the computer-implemented method of claim 9 or 10.
